# FASCICULE DE BREVET EUROPEEN

(11) **EP 0 829 462 B1**
(45) Date de publication et mention de la délivrance du brevet: **11.12.2002**
(21) Numéro de dépôt: 97202745.2
(22) Date de dépôt: 04.09.1997
(51) Int. Cl.: C07C 17/20, C07C 19/08

(54) **Procédé pour la préparation de difluorométhane**
Verfahren zur Herstellung von Difluoromethan
Process for the preparation of difluoromethane

(30) Priorité: 16.09.1996 BE 9600777
(43) Date de publication de la demande: 18.03.1998
(73) Titulaire: SOLVAY (Société Anonyme), 1050 Bruxelles (BE)
(72) Inventeur: Wilmet, Vincent, 1301 Wavre (BE); Janssens, Francine, 1800 Vilvoorde (BE)
(74) Mandataire: Jacques, Philippe

(56) Documents cités:
- EP-A- 0 732 314
- WO-A-95/35271
- WO-A-96/01241

## Description

La présente invention se rapporte à un procédé pour la préparation de difluorométhane par réaction entre du fluorure d'hydrogène et du dichlorométhane.

Les brevets US-A-2,005,711, US-A-2,749,374, US-A-2,749,375 et US-A-5,495,057 et les demandent EP-A-770588 et EP-A-767158 décrivent des procédés de préparation de difluorométhane par réaction entre du dichlorométhane et du fluorure d'hydrogène en phase liquide, en présence d'un catalyseur d'hydrofluoration. Dans ces procédés connus, le catalyseur a toutefois généralement tendance à se désactiver rapidement.
La présente invention a pour but de fournir un procédé pour la préparation du difluorométhane par réaction entre du fluorure d'hydrogène et du dichlorométhane en phase liquide, qui ne présente plus les inconvénients des procédés mentionnés ci-dessus, liés à la désactivation du catalyseur.

L'invention concerne dès lors un procédé pour la préparation de difluorométhane en continu, par réaction de dichlorométhane avec du fluorure d'hydrogène dans un milieu liquide comprenant une fraction inorganique et une fraction organique, qui se particularise par le maintien dans le milieu liquide d'une teneur pondérale en ladite fraction organique inférieure ou égale à 25 % de la somme des fractions inorganique et organique et d'une teneur pondérale en dichlorométhane inférieure ou égale à 10 % de la somme des fractions inorganique et organique.

Aux fins de la présente invention, on entend par fraction inorganique, l'ensemble des composés inorganiques présents dans le milieu liquide. La fraction inorganique comprend dès lors le fluorure d'hydrogène. Avantageusement elle comprend aussi un catalyseur d'hydrofluoration. En outre, elle peut contenir d'autres composés inorganiques, notamment du chlorure d'hydrogène et de l'eau.

La fraction organique du milieu liquide conforme au procédé selon l'invention est essentiellement constituée du dichlorométhane, du difluorométhane, des produits intermédiaires de la réaction (notamment du chlorofluorométhane) et des sous-produits éventuels de la réaction et/ou des impuretés du dichlorométhane. Dans un mode préféré de mise en oeuvre du procédé selon l'invention, le milieu liquide est constitué uniquement des fractions inorganique et organique précitées.

Par la suite, à défaut d'information contraire, les teneurs pondérales seront exprimées en % de la somme des fractions inorganique et organique précitées.

De préférence, la teneur pondérale en fraction organique est au plus égale à 20 %. De manière particulièrement préférée, elle est au plus égale à 15 %. En général, elle est au moins égale à 1 %. De préférence, elle est au moins égale à 2,5 %. De manière particulièrement préférée, elle est au moins de 5 %.

De préférence, la teneur pondérale en fraction inorganique est au moins égale à 80 %. De manière particulièrement préférée, elle est au moins égale à 85 %. En général, elle ne dépasse pas 99 % en poids. De préférence, elle ne dépasse pas 97,5 % en poids. De manière particulièrement préférée, elle ne dépasse pas 95 % en poids.

Comme exposé ci-avant, la teneur pondérale en dichlorométhane ne dépasse pas 10 %. De préférence, elle ne dépasse pas 8 %. Il est particulièrement avantageux de la maintenir inférieure ou égale à 5 %. Dans le procédé selon l'invention, la teneur pondérale en dichlorométhane est généralement d'au moins 0,05 %. Avantageusement, elle est d'au moins 0,5 %. Le plus souvent, elle est au moins égale à 1 %.

La teneur pondérale en fluorure d'hydrogène est généralement d'au moins 5 %, de préférence d'au moins 15 % et, de manière particulièrement préférée, d'au moins 30 %. Le plus souvent, elle ne dépasse pas 90 %. Avantageusement, elle ne dépasse pas 75 %. De manière particulièrement préférée, elle ne dépasse pas 65%.

La teneur pondérale en catalyseur d'hydrofluoration est habituellement d'au moins 8 %, de préférence d'au moins 15 % et, de manière particulièrement préférée, d'au moins 25 %. Le plus souvent, elle ne dépasse pas 90 %. Avantageusement, elle ne dépasse pas 75 %. De manière particulièrement préférée, elle ne dépasse pas 60 %.

Dans le cas particulier où la fraction inorganique contient, en plus du fluorure d'hydrogène et du catalyseur d'hydrofluoration, d'autres composés inorganiques (par exemple du chlorure d'hydrogène et/ou de l'eau), la teneur pondérale totale en ces autres composés inorganiques ne dépasse généralement pas 5%. Le plus souvent, elle ne dépasse pas 1 %. En particulier, la teneur pondérale en chlorure d'hydrogène ne dépasse généralement pas 2% et celle en eau ne dépasse généralement pas 0,5 %. Avantageusement, la teneur pondérale en eau est inférieure à 0,2 %.

Le catalyseur d'hydrofluoration est avantageusement choisi parmi les dérivés des métaux des groupes 3, 4, 5, 13, 14 et 15 du tableau périodique des éléments (IUPAC 1988) et leurs mélanges. On entend par dérivés des métaux, les hydroxydes, les oxydes et les sels inorganiques de ces métaux ainsi que leurs mélanges. On retient particulièrement les dérivés du titane, du niobium, du tantale, du molybdène, du bore, de l'étain et de l'antimoine. De préférence, le catalyseur est choisi parmi les dérivés des métaux des groupes 4, 14 et 15 du tableau périodique des éléments, et plus particulièrement parmi les dérivés du titane, de l'étain et de l'antimoine. Dans le procédé selon l'invention, les dérivés préférés des métaux sont leurs sels et ceux-ci sont de préférence choisis parmi les halogénures, et plus particulièrement parmi les chlorures, les fluorures et les chlorofluorures. Des catalyseurs d'hydrofluoration particulièrement avantageux dans le procédé selon l'invention sont les chlorures, les fluorures et les chlorofluorures d'étain et d'antimoine, notamment le tétrachlorure d'étain et le pentachlorure d'antimoine. Le pentachlorure d'antimoine est tout particulièrement recommandé.

Le rapport molaire entre le catalyseur et le dichlorométhane dans le milieu liquide est généralement supérieur à 1. De préférence, il est supérieur à 2. De très bons résultats ont été obtenus en présence d'au moins environ 2,5 mole de catalyseur par mole de dichlorométhane. En principe, il n'y a pas de limite supérieure à ce rapport. Il peut par exemple atteindre 1000. Le plus souvent, il ne dépasse pas 100.

Le rapport molaire entre le catalyseur et le fluorure d'hydrogène dans le milieu liquide peut varier dans de larges limites. Il est généralement supérieur à 0,01. De préférence, il est supérieur à 0,02. De très bons résultats ont été obtenus en présence d'au moins environ 0,025 mole de catalyseur par mole de fluorure d'hydrogène. En général, ce rapport ne dépasse pas 1,2. Le plus souvent, il ne dépasse pas 0,5. De bons résultats ont été obtenus avec un rapport ne dépassant pas 0,1.

Dans le procédé selon l'invention, on introduit dans le milieu liquide du fluorure d'hydrogène et du dichlorométhane, de préférence à l'état liquide et dans un rapport molaire proche de 2. En pratique, on règle ce rapport de manière à maintenir sensiblement constante la composition du milieu liquide.

Le procédé selon l'invention peut être réalisé dans de larges gammes de températures et de pressions. Généralement, on travaille à une température d'au moins environ 75 °C. Une température d'au moins environ 90 °C est préférée. Une température d'au moins environ 100 °C est particulièrement préférée. Le plus souvent, en fonction notamment de la pression admissible, cette température ne dépasse pas environ 160 °C, les températures inférieures ou égales à environ 140 °C étant spécialement recommandées. Généralement, on travaille à une pression d'au moins environ 2 bar. Une pression d'au moins environ 10 bar est préférée. Une pression d'au moins environ 15 bar est particulièrement préférée. Le plus souvent, cette pression ne dépasse pas environ 50 bar, les pressions inférieures ou égales à environ 30 bar étant spécialement recommandées.

Le procédé selon l'invention peut être mis en oeuvre dans tout type de réacteur ou d'appareillage résistant à la pression et au fluorure d'hydrogène et permettant de maintenir en permanence une composition sensiblement stable du milieu liquide. Le plus souvent, le procédé selon l'invention est réalisé dans un réacteur équipé d'un dispositif de soutirage d'un courant gazeux, par exemple dans un réacteur surmonté d'une colonne et d'un condenseur de reflux. Ce dispositif permet de maintenir en permanence une composition du milieu liquide conforme aux prescriptions exposées plus haut, par un réglage adéquat des conditions opératoires (notamment les débits des réactifs entrant dans le réacteur, la température et la pression dans le réacteur et la température dans le condenseur). En outre, une teneur pondérale en catalyseur d'hydrofluoration dans le milieu liquide au moins égale à 20 % de la somme des fractions inorganique et organique favorise une élimination aisée des composés organiques par soutirage.

Le temps de séjour des réactifs dans le réacteur doit être suffisant pour que la réaction du dichlorométhane avec le fluorure d'hydrogène se réalise avec un rendement acceptable. Il peut être déterminé aisément en fonction des conditions opératoires retenues.

Le procédé selon l'invention se déroulant en continu, il est entendu que les proportions des fractions inorganiques et organiques et les teneurs des différents constituants du milieu liquide rapportées ci-avant expriment des quantités stationnaires, obtenues après mise en régime du réacteur.

De manière surprenante, le catalyseur d'hydrofluoration se désactive beaucoup moins rapidement dans le procédé selon l'invention que dans les procédés connus, alors que la faible teneur en dichlorométhane dans le milieu liquide n'affecte pas de manière substantielle la productivité en difluorométhane.

Les exemples ci-après illustrent l'invention de manière non limitative.

### Exemple 1

Dans un autoclave de 0,5 1 en acier inoxydable HASTELLOY B2, équipé d'un agitateur à pales et surmonté d'un condenseur à double enveloppe, on a introduit 150 g de fluorure d'hydrogène et 145,8 g de tétrachlorure d'étain. L'autoclave a ensuite été plongé dans un bain thermostatisé, maintenu à une température de 130 °C, et la pression a été réglée à 23,5 bar. Le condenseur a été maintenu à une température de 45 °C. Le réacteur a été alimenté en continu en dichlorométhane et en fluorure d'hydrogène liquides, en proportions quasi stoéchiométriques, pendant 200 heures. Pendant l'essai, les débits des flux de dichlorométhane et de fluorure d'hydrogène ont été ajustés (entre 200 et 375 mmole/h pour le dichlorométhane), de manière à stabiliser le niveau de liquide dans le réacteur. L'effluent gazeux sortant en continu du condenseur a été traité dans un scrubber au moyen d'une solution d'hydroxyde de potassium de manière à abattre le fluorure d'hydrogène et le chlorure d'hydrogène qu'il contenait La composition de l'effluent gazeux ainsi traité a ensuite été déterminée par analyse en ligne par chromatographie en phase gazeuse. En régime, la teneur en fraction inorganique dans le milieu liquide dans le réacteur était environ de 87,4 % en poids, et la teneur en fraction organique était d'environ 12,6 % en poids, dont 4,9 % en poids de dichlorométhane, 5,5 % en poids de chlorofluorométhane et 1,7 % en poids de difluorométhane. L'effluent gazeux sortant du scrubber contenait en moyenne de 75 à 80 % molaire de difluorométhane, de 20 à 25 % molaire de chlorofluorométhane et moins de 2 % molaire de dichlorométhane. Globalement, 99 % du dichlorométhane mis en oeuvre a été transformé dont 78 % en difluorométhane et 22 % en chlorofluorométhane. Aucun autre produit n'a été détecté. Une productivité moyenne en difluorométhane de 0,3 mole par heure et par mole de catalyseur a été obtenue. Aucune désactivation du catalyseur n'a été observée pendant toute la durée de l'essai. Au terme des 200 heures de réaction, la productivité en difluorométhane était toujours au moins de 0,3 mole par heure et par mole de catalyseur. Par ailleurs, aucune corrosion du réacteur n'a été observée.

### Exemple 2 (comparaison)

L'essai de l'exemple 1 a été répété avec un milieu liquide initial dans le réacteur constitué de 150 g de fluorure d'hydrogène, 53 g de tétrachlorure d'étain et 100 g de dichlorométhane. Après 12 heures de réaction à 115 °C sous une pression de 23,5 bar, la teneur en fraction inorganique dans le milieu liquide dans le réacteur était environ de 69,1 % en poids et la teneur en fraction organique était d'environ 30,9 % en poids, dont 20,3 % en poids de dichlorométhane, 10,5 % en poids de chlorofluorométhane et 0,1 % en poids de difluorométhane. Après neutralisation, l'effluent gazeux sortant du condenseur contenait en moyenne 76 % molaire de difluorométhane, 22 % molaire de chlorofluorométhane et 2 % molaire de dichlorométhane. Une productivité moyenne en difluorométhane de 0,375 mole par heure et par mole de catalyseur a été obtenue pendant 30 heures. Au bout de cette période, l'activité du catalyseur a brutalement diminué, provoquant une chute rapide de la productivité en difluorométhane, de sorte que, après 40 heures de réaction, la productivité en difluorométhane était quasi nulle.

## Revendications (Revendications pour l'(les) Etat(s) contractant(s) suivant(s): DE, ES, FR, GB, IT, NL)

1. Procédé pour la préparation de difluorométhane en continu, par réaction de dichlorométhane avec du fluorure d'hydrogène dans un milieu liquide comprenant une fraction inorganique et une fraction organique, dans lequel on maintient une teneur pondérale en ladite fraction organique dans le milieu liquide inférieure ou égale à 25 % de la somme des fractions inorganique et organique et une teneur pondérale en dichlorométhane dans le milieu liquide inférieure ou égale à 10 % de la somme des fractions inorganique et organique, à l'exception d'un procédé dans lequel le milieu liquide comprend en outre du 1,1,2,2-tétrachloroéthane et la fraction inorganique comprend du SnCl₄.

2. Procédé selon la revendication 1, dans lequel la teneur pondérale en fraction organique dans le milieu liquide est de 1 à 20 % de la somme des fractions inorganique et organique.

3. Procédé selon la revendication 1 ou 2, dans lequel la teneur pondérale en fraction organique dans le milieu liquide est de 2,5 à 15 % de la somme des fractions inorganique et organique.

4. Procédé selon une quelconque des revendications 1 à 3, dans lequel la teneur pondérale en dichlorométhane dans le milieu liquide est de 0,05 à 8 % de la somme des fractions inorganique et organique.

5. Procédé selon une quelconque des revendications 1 à 4, dans lequel la teneur pondérale en fluorure d'hydrogène dans le milieu liquide est de 5 à 90 % de la somme des fractions inorganique et organique.

6. Procédé selon une quelconque des revendications 1 à 5, dans lequel la teneur pondérale en catalyseur d'hydrofluoration dans le milieu liquide est de 8 à 90 % de la somme des fractions inorganique et organique.

7. Procédé selon la revendication 6, dans lequel le catalyseur est choisi parmi les dérivés du titane, de l'étain et de l'antimoine.

8. Procédé selon la revendication 6 ou 7, dans lequel le rapport molaire entre le catalyseur et le dichlorométhane dans le milieu liquide est de 1 à 1000.

9. Procédé selon une quelconque des revendications 6 à 8, dans lequel le rapport molaire entre le catalyseur et le fluorure d'hydrogène dans le milieu liquide est de 0,01 à 1,2.

10. Procédé selon l'une quelconque des revendications 1 à 9, dans lequel on réalise la réaction à une température d'environ 75 à 160 °C et à une pression d'environ 2 à 50 bar.

## Revendications (Revendications pour l'(les) Etat(s) contractant(s) suivant(s): GR)

1. Procédé pour la préparation de difluorométhane en continu, par réaction de dichlorométhane avec du fluorure d'hydrogène dans un milieu liquide comprenant une fraction inorganique et une fraction organique, dans lequel on maintient une teneur pondérale en ladite fraction organique dans le milieu liquide inférieure ou égale à 25 % de la somme des fractions inorganique et organique et une teneur pondérale en dichlorométhane dans le milieu liquide inférieure ou égale à 10 % de la somme des fractions inorganique et organique.

2. Procédé selon la revendication 1, dans lequel la teneur pondérale en fraction organique dans le milieu liquide est de 1 à 20 % de la somme des fractions inorganique et organique.

3. Procédé selon la revendication 1 ou 2, dans lequel la teneur pondérale en fraction organique dans le milieu liquide est de 2,5 à 15 % de la somme des fractions inorganique et organique.

4. Procédé selon une quelconque des revendications 1 à 3, dans lequel la teneur pondérale en dichlorométhane dans le milieu liquide est de 0,05 à 8 % de la somme des fractions inorganique et organique

5. Procédé selon une quelconque des revendications 1 à 4, dans lequel la teneur pondérale en fluorure d'hydrogène dans le milieu liquide est de 5 à 90 % de la somme des fractions inorganique et organique.

6. Procédé selon une quelconque des revendications 1 à 5, dans lequel la teneur pondérale en catalyseur d'hydrofluoration dans le milieu liquide est de 8 à 90 % de la somme des fractions inorganique et organique.

7. Procédé selon la revendication 6, dans lequel le catalyseur est choisi parmi les dérivés du titane, de rétain et de l'antimoine.

8. Procédé selon la revendication 6 ou 7, dans lequel le rapport molaire entre le catalyseur et le dichlorométhane dans le milieu liquide est de 1 à 1000.

9. Procédé selon une quelconque des revendications 6 à 8, dans lequel le rapport molaire entre le catalyseur et le fluorure d'hydrogène dans le milieu liquide est de 0,01 à 1,2.

10. Procédé selon l'une quelconque des revendications 1 à 9, dans lequel on réalise la réaction à une température d'environ 75 à 160 °C et à une pression d'environ 2 à 50 bar.

## Claims (Claims for the following Contracting State(s): DE, ES, FR, GB, IT, NL)

1. Process for the continuous preparation of difluoromethane, by reaction of dichloromethane with hydrogen fluoride in a liquid medium comprising an inorganic fraction and an organic fraction, in which a content by weight of the said organic fraction in the liquid medium is maintained at less than or equal; to 25% of the sum of the inorganic and organic fractions and a content by weight of dichloromethane in the liquid medium is maintained at less than or equal to 10% of the sum of the inorganic and organic fractions, with the exception of a process wherein the liquid medium comprises in addition 1,1,2,2-tetrachloroethane and the inorganic fraction comprises SnCl₄.

2. Process according to Claim 1, in which the content by weight of organic fraction in the liquid medium is from 1 to 20% of the sum of the inorganic and organic fractions.

3. Process according to Claim 1 or 2, in which the content by weight of organic fraction in the liquid medium is from 2.5 to 15% of the sum of the inorganic and organic fractions.

4. Process according to any one of Claims 1 to 3, in which the content by weight of dichloromethane in the liquid medium is from 0.05 to 8% of the sum of the inorganic and organic fractions.

5. Process according to any one of Claims 1 to 4, in which the content by weight of hydrogen fluoride in the liquid medium is from 5 to 90% of the sum of the inorganic and organic fractions.

6. Process according to any one of Claims 1 to 5, in which the content by weight of hydrofluorination catalyst in the liquid medium is from 8 to 90% of the sum of the inorganic and organic fractions.

7. Process according to Claim 6, in which the catalyst is chosen from derivatives of titanium, tin and antimony.

8. Process according to Claim 6 or 7, in which the molar ratio of the catalyst to the dichloromethane in the liquid medium is from 1 to 1000.

9. Process according to any one of Claims 6 to 8, in which the molar ratio of the catalyst to the hydrogen fluoride in the liquid medium is from 0.01 to 1.2.

10. Process according to any one of Claims 1 to 9, in which the reaction is carried out at a temperature of approximately 75 to 160°C and at a pressure of approximately 2 to 50 bar.

## Claims (Claims for the following Contracting State(s): GR)

1. Process for the continuous preparation of difluoromethane, by reaction of dichloromethane with hydrogen fluoride in a liquid medium comprising an inorganic fraction and an organic fraction, in which a content by weight of the said organic fraction in the liquid medium is maintained at less than or equal to 25% of the sum of the inorganic and organic fractions and a content by weight of dichloromethane in the liquid medium is maintained at less than or equal to 10% of the sum of the inorganic and organic fractions.

2. Process according to Claim 1, in which the content by weight of organic fraction in the liquid medium is from 1 to 20% of the sum of the inorganic and organic fractions.

3. Process according to Claim 1 or 2, in which the content by weight of organic fraction in the liquid medium is from 2.5 to 15% of the sum of the inorganic and organic fractions.

4. Process according to any one of Claims 1 to 3, in which the content by weight of dichloromethane in the liquid medium is from 0.05 to 8% of the sum of the inorganic and organic fractions.

5. Process according to any one of Claims 1 to 4, in which the content by weight of hydrogen fluoride in the liquid medium is from 5 to 90% of the sum of the inorganic and organic fractions.

6. Process according to any one of Claims 1 to 5, in which the content by weight of hydrofluorination catalyst in the liquid medium is from 8 to 90% of the sum of the inorganic and organic fractions.

7. Process according to Claim 6, in which the catalyst is chosen from derivatives of titanium, tin and antimony.

8. Process according to Claim 6 or 7, in which the molar ratio of the catalyst to the dichloromethane in the liquid medium is from 1 to 1000.

9. Process according to any one of Claims 6 to 8, in which the molar ratio of the catalyst to the hydrogen fluoride in the liquid medium is from 0.01 to 1.2.

10. Process according to any one of Claims 1 to 9, in which the reaction is carried out at a temperature of approximately 75 to 160°C and at a pressure of approximately 2 to 50 bar.

## Patentansprüche (Patentansprüche für folgende(n) Vertragsstaat(en): DE, ES, FR, GB, IT, NL)

1. Verfahren zur kontinuierlichen Herstellung von Difluormethan durch Umsetzung von Dichlormethan mit Fluorwasserstoff in einem flüssigen Milieu, das eine anorganische Fraktion und eine organische Fraktion umfaßt, wobei man in dem flüssigen Milieu einen auf das Gewicht bezogenen Gehalt an der genannten organischen Fraktion von kleiner als oder gleich 25% der Summe der anorganischen und organischen Fraktionen und einen auf das Gewicht bezogenen Gehalt an Dichlormethan in dem flüssigen Milieu von kleiner als oder gleich 10% der Summe der anorganischen und organischen Fraktionen aufrechterhält, mit der Maßgabe, daß ein Verfahren ausgenommen ist, worin das flüssige Milieu zusätzlich 1,1,2,2-Tetrachlorethan umfaßt und die anorganische Fraktion SnCl₄ enthält.

2. Verfahren nach Anspruch 1, worin der auf das Gewicht bezogene Gehalt an organischer Fraktion im flüssigem Milieu 1 bis 20% der Summe der anorganischen und organischen Fraktionen beträgt.

3. Verfahren nach Anspruch 1 oder 2, worin der auf das Gewicht bezogene Gehalt an organischer Fraktion im flüssigen Milieu 2,5 bis 15% der Summe der anorganischen und organischen Fraktionen beträgt.

4. Verfahren nach einem der Ansprüche 1 bis 3, worin der auf das Gewicht bezogene Gehalt an Dichlormethan im flüssigen Milieu von 0,05 bis 8% der Summe der anorganischen und organischen Fraktionen beträgt.

5. Verfahren nach einem der Ansprüche 1 bis 4, worin der auf das Gewicht bezogene Gehalt an Fluorwasserstoff im flüssigen Milieu von 5 bis 90% der Summe der anorganischen und organischen Fraktionen beträgt.

6. Verfahren nach einem der Ansprüche 1 bis 5, worin der auf das Gewicht bezogene Gehalt an Hydrofluorierungskatalysator im flüssigen Milieu von 8 bis 90% der Summe der anorganischen und organischen Fraktionen beträgt.

7. Verfahren nach Anspruch 6, worin der Katalysator unter Verbindungen von Titan, Zinn und Antimon ausgewählt wird.

8. Verfahren nach Anspruch 6 oder 7, worin das Molverhältnis von Katalysator zu Dichlormethan im flüssigen Milieu von 1 bis 1.000 beträgt.

9. Verfahren nach einem der Ansprüche 6 bis 8, worin das Molverhältnis von Katalysator zu Fluorwasserstoff im flüssigen Milieu von 0,01 bis 1,2 beträgt.

10. Verfahren nach einem der Ansprüche 1 bis 9, worin die Umsetzung bei einer Temperatur von etwa 75 bis 160°C und bei einem Druck von etwa 2 bis 50 bar ausgeführt wird.

## Patentansprüche (Patentansprüche für folgende(n) Vertragsstaat(en): GR)

1. Verfahren zur kontinuierlichen Herstellung von Difluormethan durch Umsetzung von Dichlormethan mit Fluorwasserstoff in einem flüssigen Milieu, das eine anorganische Fraktion und eine organische Fraktion umfaßt, wobei man in dem flüssigen Milieu einen auf das Gewicht bezogenen Gehalt an der genannten organischen Fraktion von kleiner als oder gleich 25% der Summe der anorganischen und organischen Fraktionen und einen auf das Gewicht bezogenen Gehalt an Dichlormethan in dem flüssigen Milieu von kleiner als oder gleich 10% der Summe der anorganischen und organischen Fraktionen aufrechterhält.

2. Verfahren nach Anspruch 1, worin der auf das Gewicht bezogene Gehalt an organischer Fraktion im flüssigem Milieu 1 bis 20% der Summe der anorganischen und organischen Fraktionen beträgt.

3. Verfahren nach Anspruch 1 oder 2, worin der auf das Gewicht bezogene Gehalt an organischer Fraktion im flüssigen Milieu 2,5 bis 15% der Summe der anorganischen und organischen Fraktionen beträgt.

4. Verfahren nach einem der Ansprüche 1 bis 3, worin der auf das Gewicht bezogene Gehalt an Dichlormethan im flüssigen Milieu von 0,05 bis 8% der Summe der anorganischen und organischen Fraktionen beträgt.

5. Verfahren nach einem der Ansprüche 1 bis 4, worin der auf das Gewicht bezogene Gehalt an Fluorwasserstoff im flüssigen Milieu von 5 bis 90% der Summe der anorganischen und organischen Fraktionen beträgt.

6. Verfahren nach einem der Ansprüche 1 bis 5, worin der auf das Gewicht bezogene Gehalt an Hydrofluorierungskatalysator im flüssigen Milieu von 8 bis 90% der Summe der anorganischen und organischen Fraktionen beträgt.

7. Verfahren nach Anspruch 6, worin der Katalysator unter Verbindungen von Titan, Zinn und Antimon ausgewählt wird.

8. Verfahren nach Anspruch 6 oder 7, worin das Molverhältnis von Katalysator zu Dichlormethan im flüssigen Milieu von 1 bis 1.000 beträgt.

9. Verfahren nach einem der Ansprüche 6 bis 8, worin das Molverhältnis von Katalysator zu Fluorwasserstoff im flüssigen Milieu von 0,01 bis 1,2 beträgt.

10. Verfahren nach einem der Ansprüche 1 bis 9, worin die Umsetzung bei einer Temperatur von etwa 75 bis 160°C und bei einem Druck von etwa 2 bis 50 bar ausgeführt wird.
